(19) Europäisches Patentamt / European Patent Office / Office européen des brevets

(11) **EP 4 296 276 A1**

(12) **EUROPEAN PATENT APPLICATION**

(43) Date of publication:
**27.12.2023  Bulletin 2023/52**

(21) Application number: **22180453.7**

(22) Date of filing: **22.06.2022**

(51) International Patent Classification (IPC):
**C07K 7/08** *(2006.01)*     **A61K 38/04** *(2006.01)*

(52) Cooperative Patent Classification (CPC):
**C07K 7/08; A61K 38/04**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Forschungszentrum Jülich GmbH**
**52425 Jülich (DE)**

(72) Inventors:
• **CUKKEMANE, Abishek**
 **52428 Jülich (DE)**
• **WILLBOLD, Dieter**
 **52428 Jülich (DE)**

(74) Representative: **Rupprecht, Kay**
**Meissner Bolte Patentanwälte Rechtsanwälte Partnerschaft mbB**
**Kaiserswerther Straße 183**
**40474 Düsseldorf (DE)**

Remarks:
The complete document including Reference Table(s) and the Sequence Listing(s) can be downloaded from the EPO website

(54) **DISC1-BINDING PEPTIDES AND THE USE THEREOF FOR THE TREATMENT AND DIAGNOSIS OF SCHIZOPHRENIA, MAJOR DEPRESSIVE DISORDERS (MDD), AND BIPOLAR DISORDERS (BD), AND AUTISM AND OTHER CHRONIC MENTAL DISORDERS (CMDS)**

(57)     The invention relates to a peptide comprising an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, and/or SEQ ID NO: 12, and homologues, fragments and portions thereof.

EP 4 296 276 A1

**Description**

The invention relates to peptides comprising an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, and/or SEQ ID NO: 12, homologues, fragments, combinations and portions thereof, and to such a peptide for use in the diagnosis and/or treatment of chronic mental disorders, in particular schizophrenia.

Chronic mental disorders (CMDs) such as schizophrenia represent multi-factorial complex disease that is affected by an interplay of mental, biological, social and environmental conditions. With an incidence of 1%, schizophrenia represents a major social and economic burden. Taken together with related CMDs, the lifetime rate increases to 2.3 to 3.5%. The disorders represent neurodevelopmental impairments that do not manifest until decades usually during the onset of adolescence and are accompanied with significant comorbidity. A significantly rated biological risk factor that has been observed as aggregates in the post mortem brain tissue of patients suffering from schizophrenia is the protein Disrupted in Schizophrenia 1 (DISC1). However, the concept of proteinopathy in schizophrenia has remained elusive due to its microscopic nature. Recently, we demonstrated that pathophysiology of schizophrenia bears remarkable structural resemblance to other protein conformational disorder [1] akin its neurodegenerative cousins also forms β-fibrils.

Most forms of therapy that exist today are empirical and prescribed on a trial-and-error basis. They represent the 2nd and 3rd generation atypical antipsychotics that target the dopamine (D2R) and recently also seratogenic (5HT-R) receptors and primarily serve to treat symptoms (mostly psychotic) and preserve remaining abilities. Thus, it is of utmost urgency to develop an active substance that target other known important receptors, thereby target other symptoms and slow down the course of the disease.

One of the aims of the present invention was thus the selection and development of small peptides which bind with high affinity to DISC1 and/or one of the clinically identified mutant versions of DISC1 and can be used in particular as molecular tracers in the diagnosis of schizophrenia, major depressive disorders (MDD), and bipolar disorders (BD), and autism and other chronic mental disorders, and/or can be used for the treatment of schizophrenia and other chronic mental disorders.

One object of the present invention was therefore to develop new chemical entities which bind with high affinity and high specificity to DISC1 and/or one of the clinically identified mutant versions of DISC1. Such a high-affinity and highly specific binding to DISC1 and/or one of the clinically identified mutant versions of DISC1 gives the peptides according to the invention a particular suitability for the diagnosis and/or therapy of schizophrenia, MDD, and BD, and autism and other chronic mental disorders.

Another object of the present invention was therefore to develop new chemical entities which bind with a certain affinity to DISC1 and/or one of the clinically identified mutant versions of DISC1. Such a specific affinity to DISC1 and/or one of the clinically identified mutant versions of DISC1 gives the peptides according to the invention a particular suitability for the therapy and/or diagnostics of schizophrenia, MDD, and BD, and autism and other chronic mental disorders.

Another aspect of the present invention was to develop new chemical entities which bind with significantly higher affinity to clinically identified mutant versions of the DISC1 protein associated with chronic mental disorders, than to the wild type DICS1.

The present inventors have characterized 12 peptide ligands originally identified by phage display techniques. The hits were generated against the wild-type construct of the C-region of DISC1 and the identified leads bind to it as well. More importantly, the peptides bind to 3 clinically identified mutant versions of the DISC1 protein with higher affinity. These mutant versions of the C-region include S704C (observed as aggregate deposits from post-mortem brain), S713E (serves the role of a conformational switch in Bardet-Biedl syndrome) and L807-frameshift (L807-FS; identified in American family suffering from mental disorders). The S704C [2] mutant in particular is very interesting because several reports are available on its aggregation propensity, oligomer specific association with NDEL1, and this mutant has been identified among aggregates in brain tissues of major depressive disorder (MDD) and schizophrenia. The L807-frameshift [3] was first identified in an American family. This mutation results in the replacement of residues 808-854 with nine residues at the C terminus at the binding site for the NDEL1. The mutant protein is prone to severe aggregation and perhaps also may be coupled to loss of the binding to NDEL1 protein. A unique version of the C-region that is susceptible to phosphorylation is S713 [4]. In its phosphorylated state, the full-length DISC1 recruits Bardet-Biedl syndrome (BBS) proteins to the centrosome but in its dephosphorylated state interacts with GSK3β in the Wnt signalling pathway. This residue acts as switch going from proliferation (dephosphorylated state) to migration (phosphorylated state). It will be interesting to compare the atomistic structural changes between the WT-phospho-C-region against the S713E mutant [4]. This will provide us with valuable clue on the conformational switch associated with DISC1 functioning.

The peptides of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, and/or SEQ ID NO: 12, were found using optimised phage display selection. An example of the phage display selection use is described in the experimental part.

Further preferred embodiments are defined in the dependent claims. In the following, the term "comprise" shall also include "consist of".

In the conventional phage display technology, a recombinant library of randomised peptide sequences, presented at the gp3 protein of the M13 phage and encoded in its genome, is selected against the L-enantiomeric version of the target protein, i.e. the bait, which in this case is the C-region of DISC1. Thereafter, using the retro-inverso technique, the amino acid sequence of the identified lead peptides is reversed along with the inversion of chirality from L-enantiomeric amino acid to the D-enantiomer.

In the mirror image phage display, alternatively, the recombinant library of randomised peptide sequences, presented at the gp3 protein of the M13 phage and encoded in its genome, is selected against the exact mirror image (D-enantiomer) of a naturally occurring L-enantiomeric target molecule (e.g. C-region of DISC1 or the clinically identified mutant versions S704C, and/or 713E and/or L807-FS). The gp3 molecule, also called gene product 3, is a protein that sits on the coat of the phage and is required for contact with the host cell.

In the mirror image phage display, for example, a recombinant library of randomised peptide sequences, presented at the gp3 protein of the M13 phage and encoded in its genome, is selected against the exact mirror image (D-enantiomer) of a naturally occurring L-enantiomeric target molecule (e.g. C-region of DISC1 or the clinically identified mutant versions S704C, and/or 713E and/or L807-FS). The gp3 molecule, also called gene product 3, is a protein that sits in the phage's coat and is required for contact with the host cell.

The peptide sequence is advantageously presented at the N-terminus of the gp3 protein of the M13 phage and is present encoded in its genome.

The DNA sequence of the p3 gene of a selected phage is linked to the DNA sequence containing the genetic information about the corresponding peptide sequence on the gp3 molecule, allowing it to be sequenced. After sequencing, the genomic sequence can be transcribed into an amino acid sequence and synthesised as a D-enantiomeric peptide that binds to the physiological L-enantiomeric form of the target molecule (e.g. a target molecule). The totality of phages that present different peptides as a fusion protein with gp3 on their surface is referred to below as the phage library. The corresponding peptides represent the biomolecules to be selected in the experiment.

So-called panning rounds (selection rounds) can be carried out, e.g. three rounds. In this process, the phage library is brought into contact with a fixed target molecule, also called bait, and binding phages are isolated from the billion-fold background of other, non-binding phages.

In order to reduce the accumulation of phages with an affinity for plastic, L-arginine and Lysine or streptavidin, different substrate surfaces are preferably used in all panning rounds. In this case, the substrate surface is defined as chemically functionalized or biotinylated substrate used (polystyrene or polypropylene surface) and the blocking or quenching agents used. In the successive selection rounds, the selection pressure is successively increased. For this purpose, while the concentration of the target molecule remains stable, the number of washing steps after phage incubation is continuously increased from the second selection round onwards in order to remove phages that do not have an affinity for a target molecule.

Furthermore, a different substrate surface is selected in each selection round of the phage display by using other agents to block the surface after immobilisation of the target molecule on the substrate (e.g. L-arginine and Lysine, milk powder, no blocking).

By way of example, it is possible to alternate between a BSA-blocked polystyrene surface in round 1, a milk powder-blocked polypropylene surface in round 2 and a BSA-blocked polystyrene surface in round 3.

Switching between different substrate surfaces increases the specificity for the target molecule, or bait, to the surface. There is also a reduction in ligands that bind non-specifically to plastic surfaces, L-arginine and Lysine or other components of the substrate surface other than the bait molecule.

Parallel to the actual phage display selection, exemplary control selections can be carried out, which are identical to the main selection in terms of execution - with the important difference that no bait is used here. A data analysis of the sequences resulting from the control selections enables the identification of peptides that accumulate during the selection even without bait and are therefore irrelevant for all subsequent steps.

The method is thus characterised by the following steps:

a) Providing an immobilised bait on a substrate surface.
b) Contacting the immobilised molecule acting as bait with a solution containing a library of molecules to be selected.
c) Bringing the immobilised baits occupied by the molecules into contact with a washing solution.
d) Separation and multiplication of the molecules still bound to the bait after
e) contacting the immobilised bait occupied by the molecules with wash solution.
f) Repetition of the above steps, using a different substrate in each repetition,
g) Identification of the sequence of molecules remaining on the baits after repetition.

A different substrate is used, for example, by changing the type of substrate and/or blocking or not blocking it using reagents.

The bait used is a molecule selected from the group consisting of proteins, peptides, RNA, DNA, m-RNA and chemical compounds. In particular, DISC1 C-region (residues 691-836) or the clinically identified mutant versions S704C, and/or 713E and/or L807-FS can be used as bait.

The surface to which the bait is immobilised is, for example, a component from the group consisting of functionalized microtitre plates, magnetic particles, agarose or sepharose beads.

The bait according to point a) is therefore a compound to which the biomolecule to be selected is to be bound. It is fixed to a first surface according to methods known in the prior art. Exemplary, but not limiting, baits may be proteins, peptides, RNA or DNA molecules. Exemplary surfaces that may be used include functionalized microtitre plates, magnetic particles, agarose or sepharose beads. The surface with the immobilised bait can subsequently be quenched, whereby the functional groups of the substrate are inactivated. In addition, blocking of the hydrophobic free areas remaining on the substrate can be carried out with suitable agents.

In the second step b), the immobilised bait is brought into contact with a randomised library of molecules - specifically biomolecules. These biomolecules compete for binding to the bait. The randomised library is a mixture of very many, for example $10^{12}$, but also $10^4$ or only 100 different molecules in a mixture. Such a library can, for example, consist of peptides, proteins, DNA, RNA or m-RNA, which are bound to certain vehicles and which can bind to bait.

Vehicles may include, for example, phages, polysomes or bacterial surfaces. The library may comprise artificial components or components isolated from nature, or a mixture of both. Artificial in the sense of the invention means, for example, compounds produced from oligonucleotide synthesis.

The immobilised bait with biomolecules can be brought into contact with a washing substance in step c). For this purpose, a washing step is carried out in step c), in which a buffer solution is brought into contact with or rinsed with the immobilised baits. This means that the solution containing the library of biomolecules is preferably repeatedly replaced by a possibly similar or identical solution. Thus, those library molecules are removed which dissociate less rapidly from the immobilised baits than other library molecules. The speed of the dissociation reaction of the binding library molecules is mainly determined by the different dissociation constants (especially the $k_{off}$ values) of the individual molecules. Those with a small $k_{off}$ value statistically remain bound to the immobilised bait the longest and thus have a lower statistical probability of being washed away by the wash buffer. The liquid containing the wash buffer is preferably aqueous and may contain a pH buffer. Optional components of the solution for the specificity wash step may be salts, detergents or reducing agents.

After the specificity washing step in step c), the bound biomolecules are separated from the bait and amplified in step d). The separation or elution can be done, for example, by changing the pH, heating or other changes, especially increasing the salt concentration. Subsequently, the separated or eluted biomolecules are multiplied according to known methods. For this purpose, for example, phage particles obtained and eluted according to steps a) to c), which carry the biomolecules on their surface, can be introduced into cells and multiplied.

In step e), the concentration of the selected biomolecules is increased in the solution added to the bait after step a). Preferably, 3 to 6 selection rounds containing steps a) to e) are performed. However, 1 to 10 or 1 to 20 repetitions may also be carried out. Preferably, the increase in the competitor concentration in step c) with increasing number of cycles also leads to an improved selection. Preferably, an increase in the number of washing steps in step c) with an increasing number of cycles leads to an improved selection.

In this way, the following peptides were developed.

| | |
|---|---|
| SEQ ID NO 1: | IWAGHIHDWWRRGQGS-NH2 (Flm1) |
| SEQ ID NO 2: | AWQHHELKWKQYQGWR-NH2 (Flm2) |
| SEQ ID NO 3: | GIKSLISNKMHTQWNY-NH2 (Flm5) |
| SEQ ID NO 4: | WNHPMNIRSISWHKDS-NH2 (Flm6) |
| SEQ ID NO 5: | PWYNAPHSHGFWGSGR-NH2 (Flm9) |
| SEQ ID NO 6: | HKLHHLLKWENQHKVE-NH2 (Flm11) |
| SEQ ID NO 7: | FPHPTLKHREFMRHVS-NH2 (Flo1) |
| SEQ ID NO 8: | HISMQTYKWSQWAKHQ-NH2 (Flo3) |
| SEQ ID NO 9: | QDNPWWHEHKHWKSWP-NH2 (Flo5) |
| SEQ ID NO 10: | SNWHPRHFFMSPSSPQ-NH2 (Flo6) |
| SEQ ID NO 11: | KYTAIHEWYKAHNGGA-NH2 (Flo8) |
| SEQ ID NO 12: | TSGVPWWKFQQQVRRT-NH2 (Flo11) |

The task according to the invention is also solved by homologues, fragments, combinations, and parts of the amino acid sequence comprising a peptide according to SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, and/or SEQ ID NO: 12.

Sequence processing of the peptides according to the invention (e.g. sequence variation) offers the possibility of developing further substances that can be used therapeutically in the treatment of chronic mental disorders, in particular schizophrenia, MDD, and BD, and autism.

The present invention may also relate to other peptides that can be identified using the method disclosed above.

The peptides according to the invention can be used in diagnostic methods for clinical evaluation, monitoring of disease progression and early differential diagnosis of chronic mental disorders, in particular schizophrenia, MDD, and BD, and autism by specific binding to DISC1 or the clinically identified mutant versions S704C, and/or 713E and/or L807-FS).

Furthermore, the peptides according to the invention can also be used as therapeutic substances against chronic mental disorders, in particular schizophrenia, MDD, and BD, and autism.

By DISC1 or the clinically identified mutant versions S704C, and/or 713E and/or L807-FS) is preferably understood here the human DISC1 or the human form of the clinically identified mutant versions S704C, and/or 713E and/or L807-FS).

"Homologous sequences" or "homologues" means, for the purposes of the invention, that an amino acid sequence has an identity with one of the above amino acid sequences of the monomers of at least 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100%. Preferred are 80% and 90%. Instead of the term "identity", the terms "homolog" or "homology" are used synonymously in the present description. The identity between two nucleic acid sequences or 30 polypeptide sequences is determined by comparison using the program BESTFIT based on the algorithm of Smith, T. F. and Waterman, M. S (Adv. Appl. Math. 2: 482-489 (1981)) with the following parameters for amino acids: gap creation penalty: 8 and gap extension penalty: 2; and the following parameters for nucleic acids: gap creation penalty: 50 and gap extension penalty: 3. Preferably, the identity between two nucleic acid sequences or polypeptide sequences is defined by the identity of the nucleic acid sequence/polypeptide sequence over the respective entire sequence length as calculated by comparison using the program GAP based on the algorithm of Needleman, S. B. and Wunsch, C. D. (J. Mol. Biol. 48: 443-453) with the following parameters set for amino acids: Gap creation penalty: 8 and Gap extension penalty: 2; and the following parameters for nucleic acids Gap creation penalty: 50 and Gap extension penalty: 3.

Two amino acid sequences are identical within the meaning of the present invention if they have the same amino acid sequence.

In a further variant, the peptides according to the invention have sequences that differ from the indicated sequences by up to two or three amino acids.

Furthermore, sequences containing the above-mentioned sequences can also be used as peptides.

The peptide according to the invention is further preferably characterised in that an acid amide group (CONH$_2$ group) or a COH group, COCl group, COBr group, CONH-alkyl radical or a CONH-alkyl-amine radical is present at the free C-terminus instead of the carboxyl group, or else the peptide is cyclised.

In this way, the task of providing a peptide without a negative charge at the C-terminus is additionally solved in a particularly advantageous way. This has the advantage that it can bind to the target molecule with a higher affinity than a peptide that has a carboxyl group at the free C-terminus. Peptides with a free, unmodified carboxyl group have a negative charge at this end in the physiological state.

In one embodiment of the invention, the peptide according to the invention in the physiological state, in particular at pH 6-8, in particular 6.5-7.5, in particular at pH 6,0, pH 6.1, pH 6.2, pH 6.3, pH 6.4, pH 6.5, pH 6.6, pH 6.7, pH 6.8, pH 6.9, pH 7.0, pH 7.1, pH 7.2, pH 7.3, pH 7.4, pH 7.5, pH 7.6, pH 7.7, pH 7.8, pH 7.9, respectively pH 8.0 are modified in such a way that the C-terminus does not carry a negative charge, but instead is neutral or has one or more positive charges.

In one embodiment, the peptide is characterised in that an acid amide group is present at the free C-terminus instead of the carboxyl group. Instead of the carboxyl group (-COOH group), an acid amide group (-CONH2 group) is arranged at the C-terminus.

The peptide is thus particularly advantageously amidated at the free C-terminus. The peptide is thus particularly advantageously amidated at the free C-terminus and unmodified at the free N-terminus.

This solves the further problem of providing a peptide without negative excess charge, which can bind more affinely to the target molecule and is easily obtainable.

In a further embodiment, the following further groups are present in place of the carboxyl group: COH, COCl, COBr, CONH-alkyl radical, CONH-alkyl-amine radical (net positive charge), etc, although one is not limited thereto provided that the technical teaching of the main claim is followed.

In a further preferred embodiment of the invention, the affinity of binding of the peptides modified according to the invention without a negative charge at the C-terminus, compared to linear peptides with a negative charge at the C-terminus but otherwise the same amino acid sequence, is therefore increased by 1%, 2, 3, 4, 5, 6, 7, 8, 9, in particular 10%, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, especially 100%, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, especially 200%, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, in particular 300%, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, in particular 400%, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, advantageously even 500%, 501, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, particularly advantageously 600%, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 698, 699, particularly advantageously 700%, 701, 702, 703, 704, 705, 706, 707, 708, 709, 710, 711, 712, 713, 714, 715, 716, 717, 718, 719, 720, 721, 722, 723, 724, 725, 726, 727, 728, 729, 730, 731, 732, 733, 734, 735, 736, 737, 738, 739, 740, 741, 742, 743, 744, 745, 746, 747, 748, 749, 750, 751, 752, 753, 754, 755, 756, 757, 758, 759, 760, 761, 762, 763, 764, 765, 766, 767, 768, 769, 770, 771, 772, 773, 774, 775, 776, 777, 778, 779, 780, 781, 782, 783, 784, 785, 786, 787, 788, 789, 790, 791, 792, 793, 794, 795, 796, 797, 798, 799, also particularly advantageously 800%, 801, 802, 803, 804, 805, 806, 807, 808, 809, 810, 811, 812, 813, 814, 815, 816, 817, 818, 819, 820, 821, 822, 823, 824, 825, 826, 827, 828, 829, 830, 831, 832, 833, 834, 835, 836, 837, 838, 839, 840, 841, 842, 843, 844, 845, 846, 847, 848, 849, 850, 851, 852, 853, 854, 855, 856, 857, 858, 859, 860, 861, 862, 863, 864, 865, 866, 867, 868, 869, 870, 871, 872, 873, 874, 875, 876, 877, 878, 879, 880, 881, 882, 883, 884, 885, 886, 887, 888, 889, 890, 891, 892, 893, 894, 895, 896, 897, 898, 899, also particularly advantageously 900%, 901, 902, 903, 904, 905, 906, 907, 908, 909, 910, 911, 912, 913, 914, 915, 916, 917, 918, 919, 920, 921, 922, 923, 924, 925, 926, 927, 928, 929, 930, 931, 932, 933, 934, 935, 936, 937, 938, 939, 940, 941, 942, 943, 944, 945, 946, 947, 948, 949, 950, 951, 952, 953, 954, 955, 956, 957, 958, 959, 960, 961, 962, 963, 964, 965, 966, 967, 968, 969, 970, 971, 972, 973, 974, 975, 976, 977, 978, 979, 980, 981, 982, 983, 984, 985, 986, 987, 988, 989, 990, 991, 992, 993, 994, 995, 996, 997, 998, 999, or even increased by 1000%, or even increased by 10000%, or even increased by up to 100000% or 1000000%, any intermediate value being assumed.

This is indicated by a correspondingly lower $K_D$ value. The $K_D$ value as a measure of the affinity of binding of a modified peptide to the target molecule is compared to a linear binding peptide with negative charge at the free C-terminus, by 1%, 2, 99.3, 99.4, 99.5%, 99.6, 99.7, 99.8, 99.9 lowered up to 99.99 or even 99.999%, whereby any intermediate value can be assumed.

The peptide according to the invention is further preferably characterised in that it comprises 2, 3, 4, 5, 6, 7, 8, 9, 10 or more copies of the sequences having SEQ ID NO: 1 or homologues, dimers and portions thereof.

Variants are also conceivable wherein the peptide comprises 11, 12, 13, 14, 15, 16, 17, 18, 19, 20 or more peptides having SEQ ID NO: 1 or homologues, dimers and portions thereof.

Particularly preferred are dimers of the sequence containing SEQ ID NO: 1 or homologues, dimers and parts thereof.

In one embodiment, the peptide according to the invention is further characterised in that the peptide consists essentially of D-amino acids.

For the purposes of the present invention, the term "consisting essentially of D-enantiomeric amino acids" means that the monomers to be used according to the invention are composed of at least 50%, 55%, 60%, 65%, 70% preferably 75%, 80%, particularly preferably 85%, 90%, 95%, especially 96%, 97%, 98%, 99%, 100% of D-enantiomeric amino acids.

In one embodiment, the peptide according to the invention is further characterised in that the peptide consists essentially of L-amino acids.

For the purposes of the present invention, the term "consisting essentially of L-enantiomeric amino acids" means that the monomers to be used according to the invention are composed of at least 50%, 55%, 60%, 65%, 70% preferably 75%, 80%, particularly preferably 85%, 90%, 95%, especially 96%, 97%, 98%, 99%, 100% of L-enantiomeric amino acids.

The peptide according to the invention is further preferably characterised in that it consists of an amino acid sequence according to SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, and/or SEQ ID NO: 12, or homologues, dimers and parts thereof.

The peptide according to the invention is further preferably characterised in that the peptide is linked to a further substance, in particular to a labelling substance, preferably selected from the group consisting of fluorescent tags, radioactive tags, and isotope labelled groups at various positions in the amino acids.

For the purposes of the invention, the linkage is a chemical bond as defined in Römpp Chemie Lexikon, 9th edition, volume 1, page 650 ff, Georg Thieme Verlag Stuttgart, preferably a principal valence bond, in particular a covalent bond.

In an alternative, the linked substances are medicinal products or active substances, defined according to the Medicinal Products Act §2 or §4 (19). §In an alternative, active substances are therapeutically active substances that are used as medicinally active substances.

In another variant, the substances are compounds that enhance the effect of the peptides.

In another alternative, these are compounds that improve the solubility of the peptides and/or the passage of the blood-brain barrier.

In an alternative, the peptides according to the invention have any combination of at least two or more features of the variants, embodiments and/or alternatives described above.

The peptide according to the invention is further preferably characterised in that several peptides of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, and/or SEQ ID NO: 12, or homologues, dimers and parts thereof are covalently or non-covalently linked to each other.

For the purposes of the invention, a covalent linkage of the peptide units is present if the peptides are linked linearly head to head, tail to tail or head to tail, with or without interposed linkers or linker groups.

The peptide according to the invention is further preferably characterised in that several peptides of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, and/or SEQ ID NO: 12, or homologues, dimers and parts thereof are linked to each other without a linker, i.e. directly to each other, or are linked to each other with a linker group.

A non-covalent linkage in the sense of the invention is present if the peptides are linked to each other, for example, via biotin and streptavidin, in particular streptavidin tetramer.

The peptide according to the invention is further preferably characterised in that several peptides of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, and/or SEQ ID NO: 12, or homologues, dimers and parts thereof are linked together near or branched.

In one variant of the present invention, the peptides may be linked linearly to one another, in particular as described above. In another variant, the peptides are linked together in a branched manner to form the peptide according to the invention.

According to the invention, a branched peptide may be a dendrimer in which the monomers are covalently or non-covalently linked to each other.

Alternatively, the peptides can be linked to a platform molecule (such as PEG or sugar) to form a branched peptide.

Alternatively, combinations of these options are also possible.

In one embodiment of the invention, the affinity of the binding of the peptides is defined by the dissociation constant ($K_D$ value).

The dissociation constant ($K_D$ value) of a peptide according to the invention is thereby advantageously reduced in an advantageous embodiment of the invention. This is associated with improved properties of the peptides according to the invention, such as higher affinity for binding to DISC1 or the clinically identified mutant versions S704C, and/or 713E and/or L807-FS.

In one variant of the invention, peptides are used which are bound to a DISC1 protein with a dissociation constant ($K_D$-value) of at most 500 $\mu$M, preferably 250, 100, 50 $\mu$M, particularly preferably 25, 10, 1 $\mu$M, whereby any

intermediate value can be assumed.

The peptides according to the invention are further preferably characterised in that they bind to the DISC1 C-region (residues 691-836) protein with a $K_D$ of less than 100 $\mu$M, in particular less than 10 $\mu$M, in particular 100 $\mu$M to 0,01 $\mu$M, more preferably 10 $\mu$M to 0,05 $\mu$M.

In one variant of the invention, peptides are used which are bound to the clinically identified DISC1 C-region (residues 691-836) mutant versions S704C, and/or 713E and/or L807-FS with a dissociation constant ($K_D$-value) of at most 50 $\mu$M, preferably 25, 10, 5 $\mu$M, particularly preferably 2,5, 1, 0,1 $\mu$M, whereby any intermediate value can be assumed.

The peptides according to the invention are further preferably characterised in that they bind to the clinically identified DISC1 C-region (residues 691-836) mutant versions S704C, and/or 713E and/or L807-FS with a $K_D$ of less than 10 $\mu$M, in particular less than 1 $\mu$M, in particular 10 $\mu$M to 0,001 $\mu$M, more preferably 1 $\mu$M to 0,005 $\mu$M.

The peptides according to the invention are further preferably characterised in that they bind to the clinically identified DISC1 C-region (residues 691-836) mutant versions S704C, and/or 713E and/or L807-FS with a KD of less than 10 nM, in particular less than 5 nM, in particular 1 nM to 5 pM.

The peptides can be produced, for example, via chemical synthesis or peptide synthesis or by recombinant methods.

The present invention also relates to the peptide described above for use as a probe for the identification, quantitative and/or qualitative determinations of DISC1 or the clinically identified mutant versions S704C, and/or 713E and/or L807-FS.

However, the peptides according to the invention can also be used as therapeutic agents, in particular for the treatment of chronic mental disorders, in particular schizophrenia, MDD, and BD, and autism.

The present invention thus also relates to the peptides described above for use in the treatment of chronic mental disorders, in particular schizophrenia, MDD, and BD, and autism.

Furthermore, the present invention also relates to the use of the above-described peptides as a probe for the identification, quantitative and/or qualitative determination of DISC1 and/or the clinically identified mutant versions S704C, and/or 713E and/or L807-FS, in particular as a tracer in an imaging method for clinical evaluation and/or monitoring of disease progression and/or for the early differential diagnosis of chronic mental disorders, in particular schizophrenia, MDD, and BD, and autism.

## Description of the figures

Fig. 1: A schematic representation of the phage display technique. Two parallel selection rounds were performed to enrich peptide displaying phages during the TS1 and ES1. Direct controls (DC2 and DC3) were utilized as negative controls sequences, which were compared with target selection sequences after NGS sequencing to value which peptides were not target specific.

Fig. 2: Sequences selected on WT C-region ranked by empty score. The first 10 sequences (Flm, where m = monomer) were further investigated.

Fig. 3: Sequences selected on WT C-region ranked by empty score. The first 10 sequences (Flo, where o = oligomer) were further investigated.

Fig. 4: BLI response featuring binding of Flm1 (IWAGHIHDWWRRGQGS-NH2) with C-region constructs of DISC1 and clinically identified mutant versions of DISC1.

Fig. 5: BLI response featuring binding of Flm2 (AWQHHELKWKQYQGWR-NH2) with C-region constructs of DISC1 and clinically identified mutant versions of DISC1.

Fig. 6: BLI response featuring binding of Flm5 (GIKSLISNKMHTQWNY-NH2) with C-region constructs of DISC1 and clinically identified mutant versions of DISC1.

Fig. 7: BLI response featuring binding of Flm6 (WNHPMNIRSISWHKDS-NH2) with C-region constructs of DISC1 and clinically identified mutant versions of DISC1.

Fig. 8: BLI response featuring binding of Flm9 (PWYNAPHSGFWGSGR-NH2) with C-region constructs of DISC1 and clinically identified mutant versions of DISC1.

Fig. 9:    BLI response featuring binding of Flm11 (HKLHHLLKWENQHKVE-NH2) with C-region constructs of DISC1 and clinically identified mutant versions of DISC1.

Fig. 10:    BLI response featuring binding of Flo1 (FPHPTLKHREFMRHVS-NH2) with C-region constructs of DISC1 and clinically identified mutant versions of DISC1.

Fig. 11:    BLI response featuring binding of Flo3 (HISMQTYKWSQWAKHQ-NH2) with C-region constructs of DISC1 and clinically identified mutant versions of DISC1.

Fig. 12:    BLI response featuring binding of Flo5 (QDNPWWHEHKHWKSWP-NH2) with C-region constructs of DISC1 and clinically identified mutant versions of DISC1.

Fig. 13:    BLI response featuring binding of Flo6 (SNWHPRHFFMSPSSPQ-NH2) with C-region constructs of DISC1 and clinically identified mutant versions of DISC1.

Fig. 14:    BLI response featuring binding of Flo8 (KYTAIHEWYKAHNGGA-NH2) with C-region constructs of DISC1 and clinically identified mutant versions of DISC1.

Fig. 15:    BLI response featuring binding of Flo11 (TSGVPWWKFQQQVRRT-NH2) with C-region constructs of DISC1 and clinically identified mutant versions of DISC1.

Fig. 16:    Binding affinities of the 12 different peptide hits with the C-region constructs of DISC1 and clinically identified mutant versions of DISC1.

**Material and Methods**

**Protein expression and purification.** The wild-type (WT) human DISC1 C-region (residues 691-836) was expressed alternatively as N-terminal His$_6$ - fusion construct, which was expressed as previously described [5] using the E. coli BL21 (DE3) pLysE T1R cells. The culture was grown either in Luria-Bertani (LB) broth and protein expression was induced at an $OD_{600}$ of 0.6 by the addition of 1 mM isopropyl-$\beta$-D-thiogalactopyranoside (IPTG) for 16 h at 18 °C. Harvested cells were lysed using an ice-chilled microfluidizer M100P (Microfluidics MPT) at 15, 000 PSI in Tris-buffered saline (TBS) buffer containing 10 mM Tris-HCl pH 7.4, 150 mM NaCl and Complete EDTA- free protease inhibitor cocktail (Roche). The soluble fraction was purified on $Co^{2+}$-NTA resin (Qiagen) and was eluted using TBS containing 500 mM imidazole. In the next step, the WT C-region protein was further purified on a HiLoad 16/60 Superdex 200 size exclusion chromatography column (GE Healthcare Bio Sciences).

**Phage display technique for identification of peptide hits.** Freshly prepared 100 $\mu$g/ml of the monomeric and 15 mg/ml of a week-old oligomeric WT proteins were used as the targets for the phage display selection included the monomeric and the oligomeric versions of the above-mentioned protein, which was immobilized on 3D-NHS Matrix plates (PolyAn molecular surface engineering). During the phage display selection experiment multiple control selections were performed to ensure the quality of the selection. From the library (TriCo16, BioLabs) two starting selections were performed. The first, called TS1 for target selection 1, used a surface that contained the target, while the second one, called ES1 for empty selection 1, did not contain the target on the surface and served as a control. The empty selection was performed in parallel to the target selection. In the first round of selection, the wells were blocked with 22 mg/ml of L-Arginine.

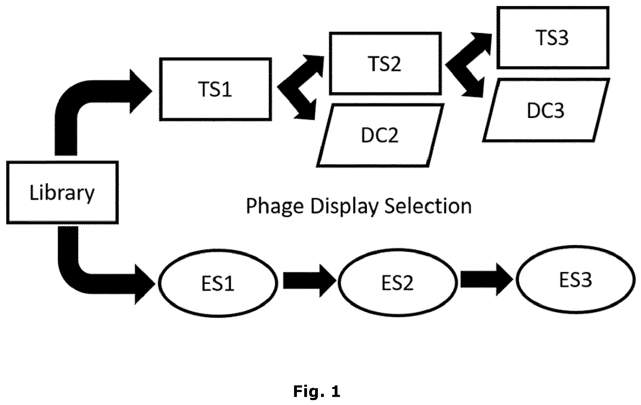

The phages derived from TS1 were later applied to the next target selection (TS2) as well as the direct control (DC2) which did not contain the target protein on the surface and served as a negative control for direct comparison. In the next round of selection, the wells were blocked with 15 mg/ml of L-Lysine. A subsequent third selection round was performed analogously and the wells were blocked with 22 mg/ml of L-Arginine. Figure 1 illustrates this course of the selection and at which points a control selection had been performed which subsequently enables the identification of target specific binders via next-generation sequencing (NGS) results. The different selection types were named "target selection" (TS), "direct control" (DC) and "empty selection" (ES). Next generation sequencing (NGS) of the phage DNA was performed by the Genomics & Transcriptomics Laboratory of the Heinrich-Heine-Universitat Düsseldorf. For NGS all target selection inputs as well as direct control input three and empty selection input three were prepared and sequenced, this included a sample of the library.

**Data analysis for identification of peptide hits.** For the initial analysis and grouping of the NGS data using an in-house developed program TSAT (Target Sequence Analysis Tool). A secondary analysis was performed using the clustering software Hammock [6]. The program TSAT takes raw DNA data in a Fasta or Fastq format as an input and examines this data for the provided framing regions of the randomized peptide region. If the framing regions are present in the given DNA the randomized region coding for the peptide is excised, grouped, translated into amino acids and saved in a database. The saved sequences are further processed by filtering, sequence frequency of the last target selection round is analyzed for their enrichment from target round to target round as well as compared to empty selection and direct control. The filtering is performed in the following order:

*TS3 ≥ ES3 & TS3 ≥ DC3 & TS3 ≥ TS2 & TS2 ≥ ES2 & TS2 ≥ DC2 & TS2 ≥ TS1 & TS1 ≥ ES1& TS1 ≥ Library*

Each sequence is assigned an enrichment score and an empty score. These are based on the ratio between the last target selection round, the library and the last empty selection round. The scores are calculated according to the following formulas:

$$Enrichment\ score\ = \frac{(Frequency\ of\ a\ sequence\ in\ TS3)}{(Frequency\ of\ the\ same\ sequence\ in\ library)}$$

$$Empty\ score\ = \frac{(Frequency\ of\ a\ sequence\ in\ TS3)}{(Frequency\ of\ the\ same\ sequence\ in\ ES3)}$$

**Bio-layer interferometry (BLI) for identification of peptide leads.** Binding of the peptides with the C-region constructs (WT, S713E, S704C and L807-FS) were investigated using an Octet (ForteBio) instrument. Prior to measurements, all peptides were solubilized in phosphate buffered saline (PBS) containing 20 mM Na-phosphate, pH 7.4, and 150 mM NaCl. For coupling of the peptide (ligand), the A2RG sensor chip (Sartorius GmbH) were activated with 1-ethyl-3-(3-dimethylaminopropyl) carbodiimide (EDC)/N hydroxysuccinimide (NHS) (200 mM/50 mM); the peptides were diluted to 2 $\mu$M in 10 mM sodium acetate, pH 4.0, and immobilized to the sensor chip, and the flow cell was deactivated with 0.1 M ethanolamine-HCl, pH 8.5. Titrations of the above-mentioned C-region and the constructs were performed at 25 °C with TBS, pH 7.4 as the running buffer using protein concentrations of 2 $\mu$M without intermittent regeneration.

**Data analysis of peptide-protein interaction.** In order to assess the binding isotherms of the protein to the immobilized peptides, I derived the binding affinity ($K_D$) by computing the on-rate ($K_{on}$) and the off-rate ($K_{off}$) by deriving the reciprocals of the exponential fittings of association and dissociation (OriginPro 2020).

$$Association\ time \to y$$
$$= y0\ +\ A1 * (1 - \exp\left(-\frac{x}{t1}\right)) + A2 * (1 - \exp\left(-\frac{x}{t2}\right))$$

$$Dissociation\ time \to\ y\ =\ y0\ +\ A1 * exp\left(-\frac{x}{t1}\right) + A2 * exp\left(-\frac{x}{t2}\right)$$

The basic reaction describing the association/dissociation of the peptide-protein interaction can be represented as

$$[Peptide] + [Protein] \rightleftharpoons [Peptide - Protein]_{complex}$$

$$K_D = \frac{[Peptide - Protein]_{complex}}{[Peptide][Protein]} = \frac{k_{off}}{k_{on}}$$

The binding affinity ($K_D$) was derived by dividing the off-rate ($K_{off}$) with the on-rate ($K_{on}$).

**Results**

**Peptide selection and next generation sequencing.** Our activity pertains identifying therapeutics for the protein risk factor associated in schizophrenia *viz.* Disrupted in Schizophrenia 1 (DISC1) using phage display technology (Figure 1). Most forms of therapy that exist today are empirical and prescribed on a trial- and-error basis. They represent the 2nd and 3rd generation atypical antipsychotics that target the Dopmaine (D2R) and recently also seratogenic (5HT-R) receptors and primarily serve to treat symptoms (mostly psychotic) and preserve remaining abilities. Therefore, it is essential to find atypical binding partners of the monomeric and oligomeric atypical target proteins. DISC1 mutant proteins have been clinically identified in the families as well as post-mortem tissues of schizophrenic and MDD patients [7]. Recently our group has demonstrated the atomistic details of the architecture of the oligomers and the fibrils, which show remarkable resemblance to other proteinopathies in neurodegenerative diseases.

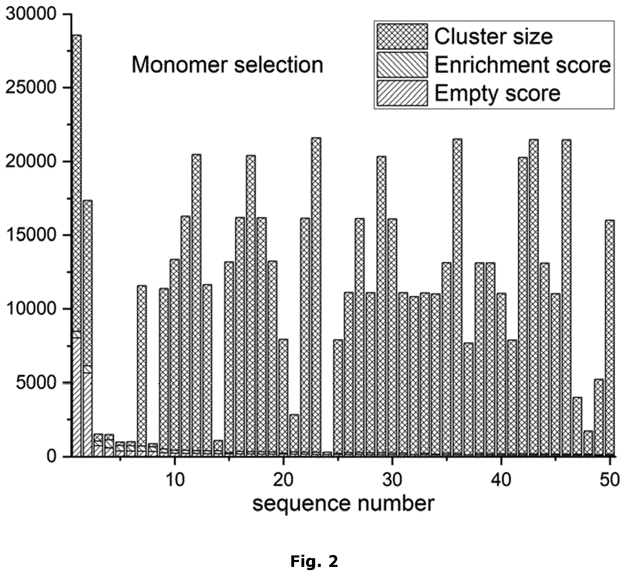

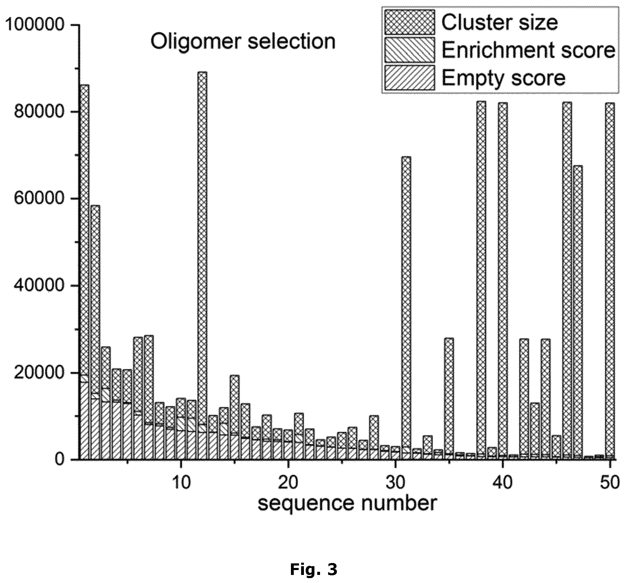

To this end, we identified several binding partners (17mer peptides) for the monomeric (Figure 2) and the oligomeric (Figure 3) target WT C-region of the DISC1 protein. To identify the peptide hits, NGS was performed, which has the ability to sequence millions of phage genomes coding for peptides presented on the phages. As mentioned above, using TSAT and HAMMOCK several rounds of filtering was performed and compared with the empty selection and direct control. Sequences that were enriched from one selection round to another and were more frequent than ES and DC pass the filter. Finally, the empty and enrichment scores (see methodology, Figures 2 and 3) were calculated for the filtered sequences and the peptides were rated largely on the basis of their empty and enrichment scores as well as the size of the generated clusters. Overall 10 sequences were identified against the monomeric and oligomeric targets, respectively. These sequences exhibited a high enrichment and empty scores and formed large cluster of similar sequences following Hammock analysis. Only sequences with an empty and enrichment score of above 1,000 and 8,000 for the monomeric and oligomeric targets, respectively were considered for further testing using BLI to study peptide-protein interactions.

**Protein-peptide interactions.** The interaction of WT C-region of DISC1 was studied using BLI, where the identified 17mer peptides were immobilized to the sensor chip. I chose to study three different mutant preparations of the DISC1 C-region along with *viz.* S713E, S704C and L807-FS, in order to assess potential of the peptides for identifying clinically relevant targets. From this set of experiments, we identified 12 potential peptides that bind to the C-region and the mutant versions of the protein:

    Flm1 -> IWAGHIHDWWRRGQGS-NH2
    Flm2 -> AWQHHELKWKQYQGWR-NH2
    Flm5 -> GIKSLISNKMHTQWNY-NH2
    Flm6 -> WNHPMNIRSISWHKDS-NH2
    Flm9 -> PWYNAPHSHGFWGSGR-NH2
    Flm11 -> HKLHHLLKWENQHKVE-NH2
    Flo1 -> FPHPTLKHREFMRHVS-NH2
    Flo3 -> HISMQTYKWSQWAKHQ-NH2
    Flo5 -> QDNPWWHEHKHWKSWP-NH2
    Flo6 -> SNWHPRHFFMSPSSPQ-NH2
    Flo8 -> KYTAIHEWYKAHNGGA-NH2
    Flo11 -> TSGVPWWKFQQQVRRT-NH2

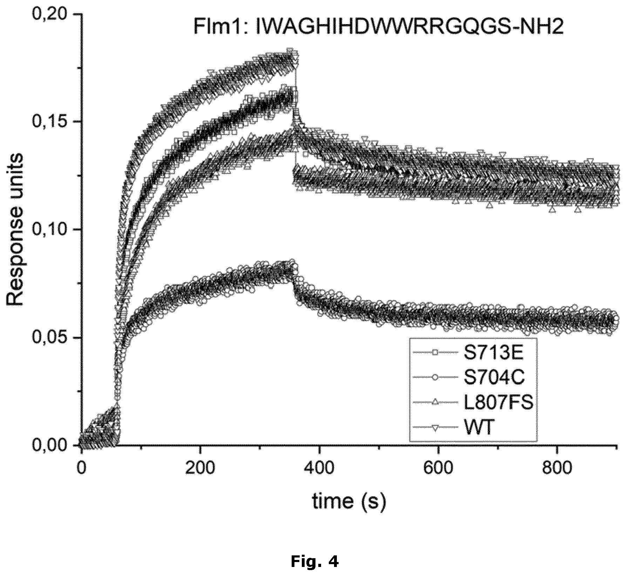

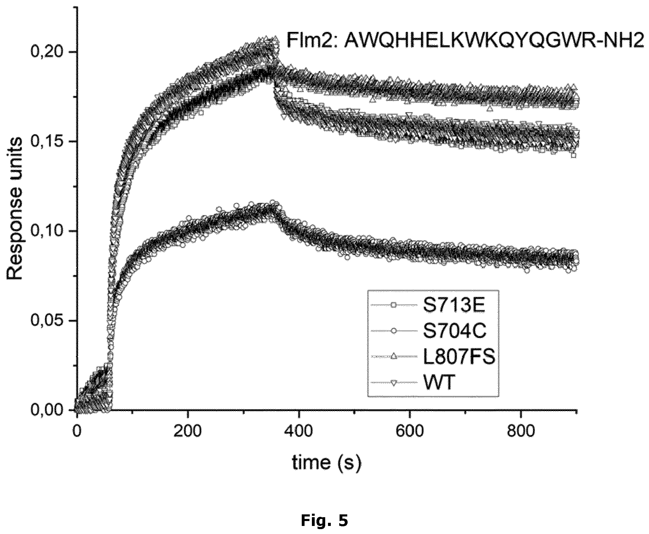

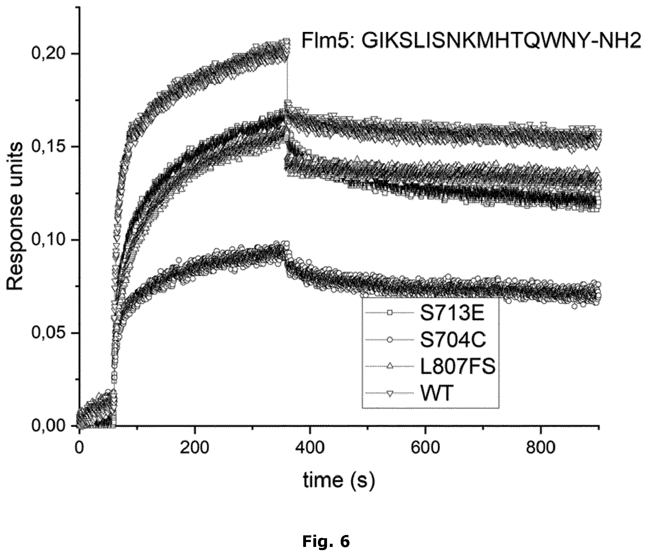

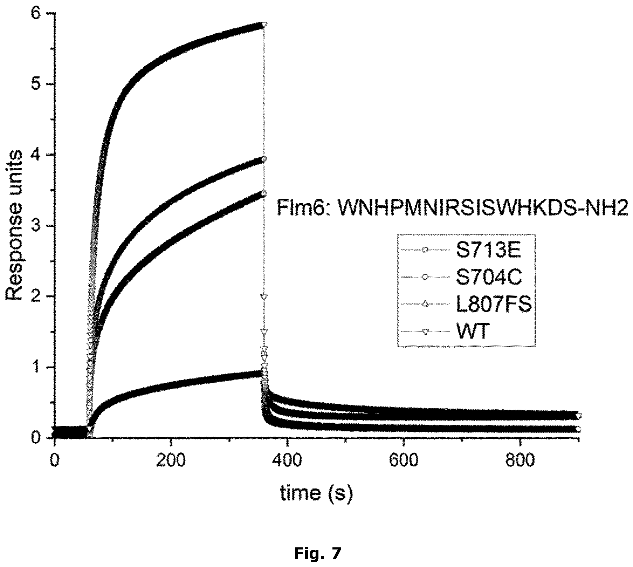

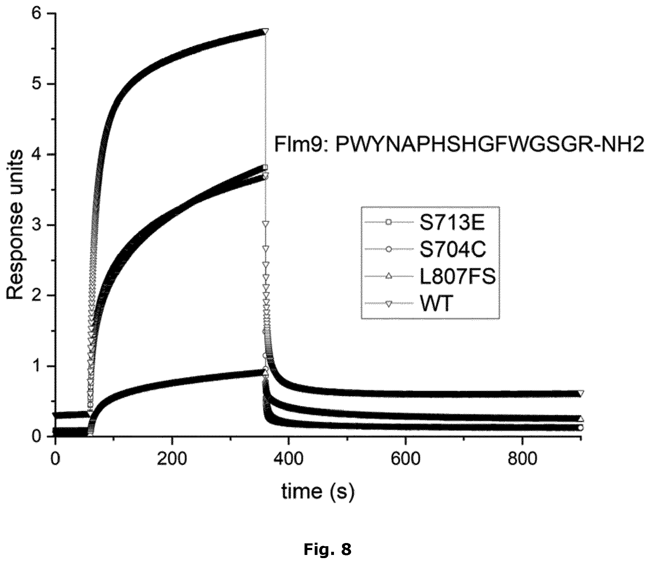

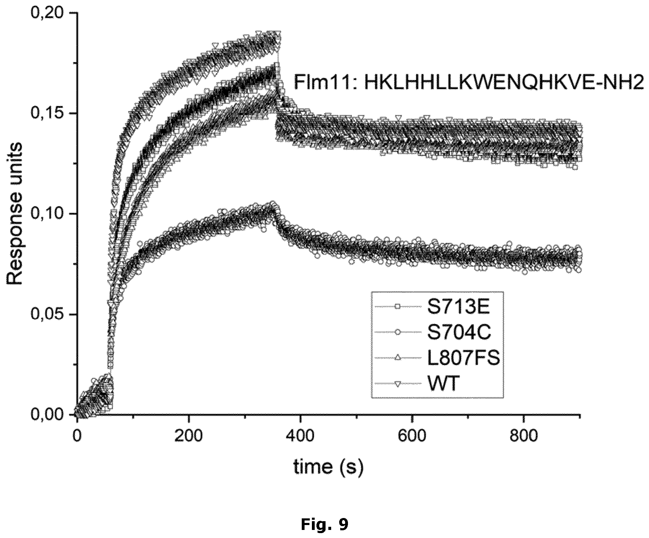

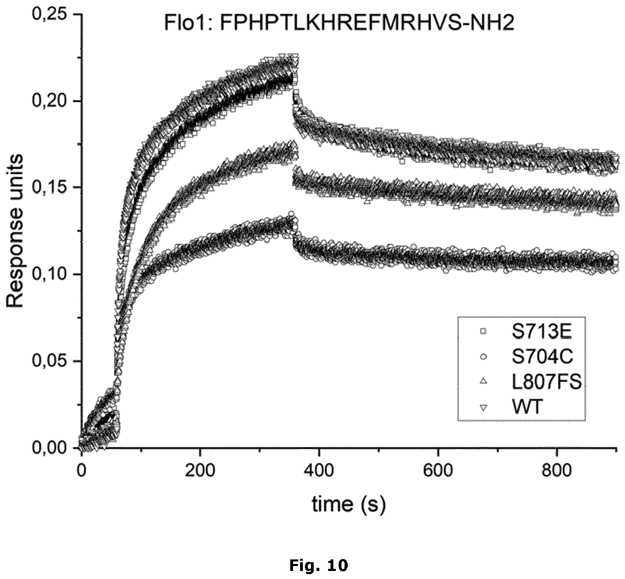

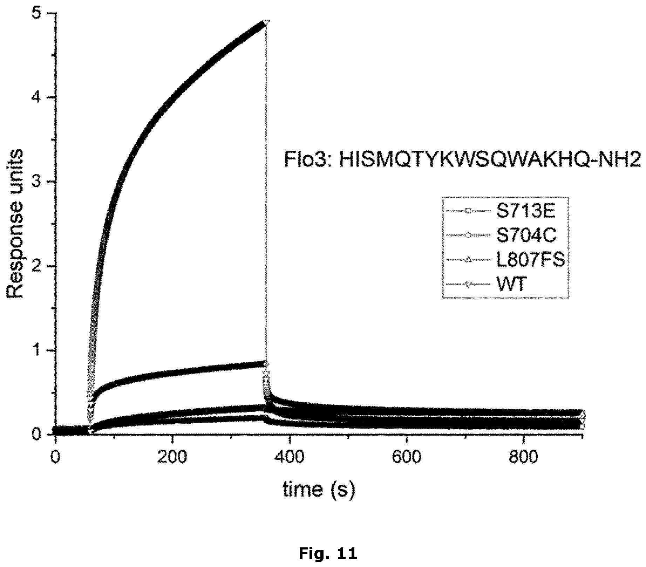

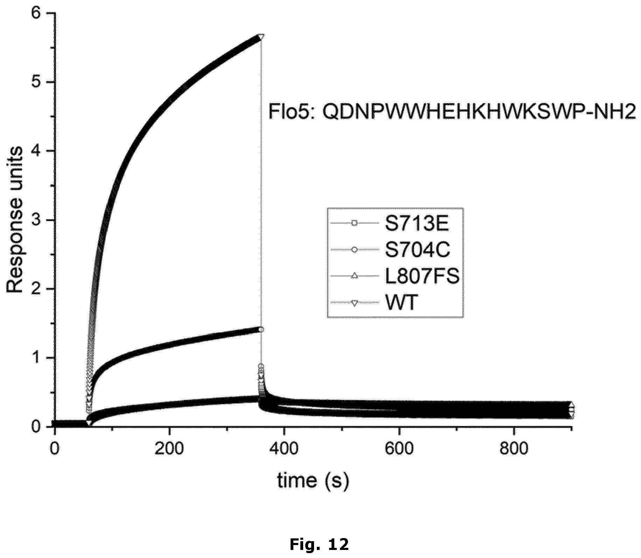

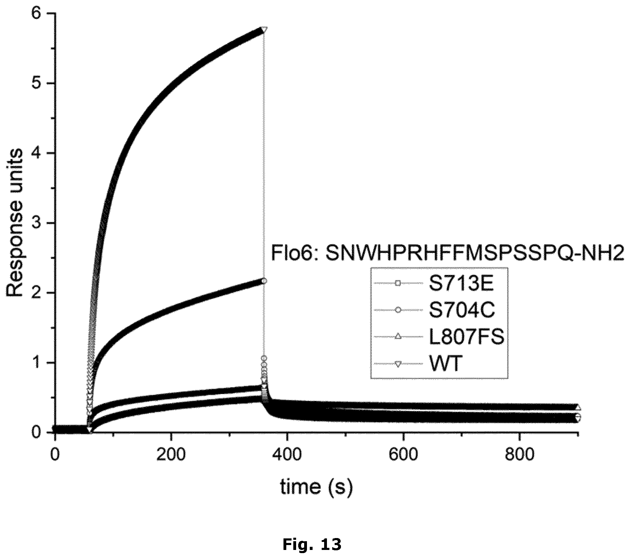

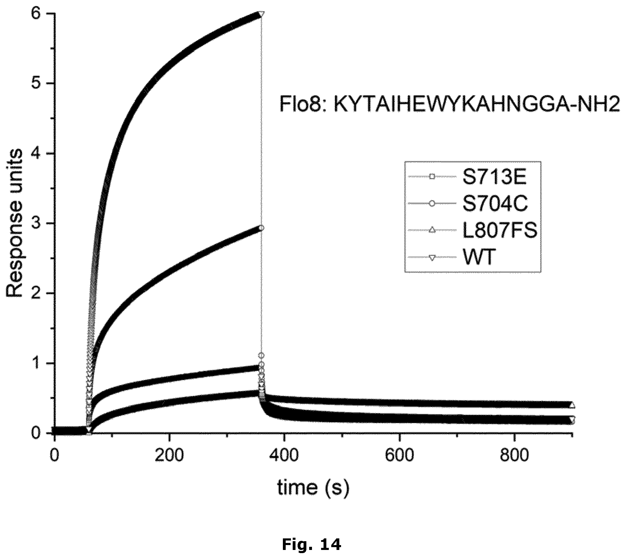

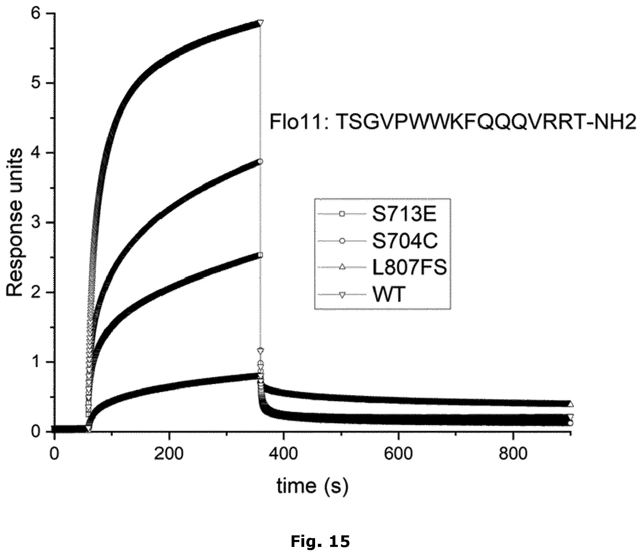

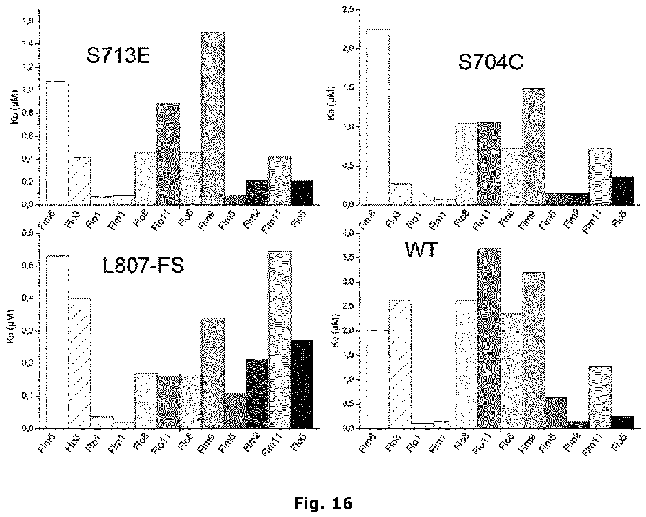

The binding profiles of the peptides to the C-region constructs are shown in the Figures 4-15 for Flm1 (Figure 4), Flo1 (Figure 5), Flm5 (Figure 6), Flm6 (Figure 7), Flm9 (Figure 8), Flm11 (Figure 9), Flo1 (Figure 10), Flo3 (Figure 11), Flo5 (Figure 12), Flo6 (Figure 13), Flo8 (Figure 14), and Flo11 (Figure 15). Most of the peptides displayed low $\mu$M affinity for the WT C-region and nM affinities for the mutant versions (Table 1 and Figure 16). The lowest $K_D$ values were observed for the L807-FS mutant.

Table 1: Summary of binding constants

| | Flm6 ($K_D$ $\mu$M) | Flo3 ($K_D$ $\mu$M) | Flo1 ($K_D$ $\mu$M) | Flm1 ($K_D$ $\mu$M) | Flo8 ($K_D$ $\mu$M) | Flo11 ($K_D$ $\mu$M) |
|---|---|---|---|---|---|---|
| S713E | 1.07 | 0.42 | 0.07 | 0.08 | 0.46 | 0.89 |
| S704C | 2.24 | 0.27 | 0.16 | 0.08 | 1.04 | 1.06 |
| L807-FS | 0.53 | 0.40 | 0.04 | 0.20 | 0.17 | 0.16 |
| WT | 2.01 | 2.62 | 0.10 | 0.15 | 2.62 | 3.68 |
| | | | | | | |
| | Flo6 ($K_D$ $\mu$M) | Flm9 ($K_D$ $\mu$M) | Flm5 ($K_D$ $\mu$M) | Flm2 ($K_D$ $\mu$M) | Flm11 ($K_D$ $\mu$M) | Flo5 ($K_D$ $\mu$M) |
| S713E | 0.46 | 1.50 | 0.08 | 0.22 | 0.42 | 0.21 |
| S704C | 0.72 | 1.49 | 0.15 | 0.15 | 0.72 | 0.36 |

(continued)

|  | Flo6 ($K_D$ μM) | Flm9 ($K_D$ μM) | Flm5 ($K_D$ μM) | Flm2 ($K_D$ μM) | Flm11 ($K_D$ μM) | Flo5 ($K_D$ μM) |
|---|---|---|---|---|---|---|
| L807-FS | 0.17 | 0.39 | 0.11 | 0.11 | 0.54 | 0.27 |
| WT | 2.36 | 3.20 | 0.64 | 0.64 | 1.27 | 0.25 |

**Efficacy.** The results suggest that the identified peptides bind to the WT C-region of the DISC1 protein with an affinity in the low micromolar range. This is critical because the peptides can be used as therapeutic agents against DISC1, which is a scaffold protein that interacts with over 300 different proteins and is required for several physiological process. If the binding affinity is too high, then it may prevent other interaction proteins to bind and lead to undesirable effects. Hence, the peptides can serve as a therapeutic agent against aggregation of the WT DISC1 protein.

On the other hand, most of the peptides display 10-fold and higher affinity for the mutant versions of the protein. This provides a facile diagnostic tool for identification of DISC1 related schizophrenia. As the entire diagnosis and therapy structure in schizophrenia and related chronic mental illnesses is empirical, identifying DISC1 anomaly would be critical for further treatment. The solution presented here is based on a risk factor biomarker i.e. the DISC1 protein, which has been observed as aggregated protein in 20-25% of post-mortem brain tissues [7] from samples involving schizophrenia, MDD and BD. One such application would involve an affinity chromatography procedure wherein a peptide among the 12 is bound to the column and the DISC1 mutant is isolated from biological fluid (blood and/or CSF) sample.

**Conclusion**

The itinerary of the current invention is two-pronged for the various lead peptides that we have identified. On one hand the high affinity peptides can be used to capture DISC1 proteins from blood and/or CSF as a diagnostic marker.

**References**

1. Cukkemane A, Becker N, Zielinski M, Frieg B, Lakomek NA, Heise H, Schroder GF, Willbold D, Weiergräber OH (2021) Conformational heterogeneity coupled with beta-fibril formation of a scaffold protein involved in chronic mental illnesses. Transl. Psychiatry 11:639

2. Hashimoto R, Numakawa T, Ohnishi T, Kumamaru E, Yagasaki Y, Ishimoto T, Mori T, Nemoto K, Adachi N, Izumi A, Chiba S, Noguchi H, Suzuki T, Iwata N, Ozaki N, Taguchi T, Kamiya A, Kosuga A, Tatsumi M, Kamijima K, Weinberger DR, Sawa A, Kunugi H (2006) Impact of the DISC1 Ser704Cys polymorphism on risk for major depression, brain morphology and ERK signaling. Hum Mol Genet. 15:3024-3033.

3. Sachs NA, Sawa A, Holmes SE, Ross CA, DeLisi LE, Margolis RL (2005) A frameshift mutation in Disrupted in Schizophrenia 1 in an American family with schizophrenia and schizoaffective disorder. Mol Psychiatry. 10:758-764.

4. Ishizuka K, Kamiya A, Oh EC, Kanki H, Seshadri S, Robinson JF, Murdoch H, Dunlop AJ, Kubo K-I, Furukori K, Huang B, Zeledon M, Hayashi-Takagi A, Okano H, Nakajima K, Houslay MD, Katsanis N, Sawa A (2011) DISC1-dependent switch from progenitor proliferation to migration in the developing cortex. Nature 473:92-96.

5. Yerabham ASK, Mas PJ, Decker C, Soares DC, Weiergräber OH, Nagel-Steger L, Willbold D, Hart DJ, Bradshaw NJ and Korth C (2017) A structural organization for the Disrupted in Schizophrenia 1 protein, identified by high-throughput screening, reveals distinctly folded regions, which are bisected by mental illness-related mutations. J. Biol. Chem. 292:6468-6477.

6. Krejci A, Hupp TR, Lexa M, Vojtesek B and Muller P (2016) Hammock: a hidden Markov model-based peptide clustering algorithm to identify protein-interaction consensus motifs in large datasets. Bioinformatics. 32:9-16.

7. Korth C. Aggregated proteins in schizophrenia and other chronic mental diseases: DISC1opathies. Prion. 2012;6(2): 134-41.

**Claims**

1. A peptide comprising an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, and/or SEQ ID NO: 12, and homologues, combinations, fragments and portions thereof.

2. A peptide according to claim 1, **characterized in that** the peptide comprises an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, and/or SEQ ID NO: 12, or homologues having an identity of at least 80% thereof.

3. A peptide according to any one of the preceding claims, **characterised in that** an acid amide group ($CONH_2$ group) or a COH group, COCl group, COBr group, CONH-alkyl radical or a CONH-alkyl-amine radical is present at the free C-terminus instead of the carboxyl group, or else the peptide is cyclised.

4. A peptide according to any one of the preceding claims, **characterized in that** it contains 2, 3, 4, 5, 6, 7, 8, 9, 10 or more copies of the sequences having SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, and SEQ ID NO: 12.

5. A peptide according to any one of the preceding claims, **characterised in that** the peptide consists essentially of D-amino acids.

6. A peptide according to any one of the preceding claims, **characterized in that** the peptide consists of an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, and SEQ ID NO: 12.

7. A peptide according to any one of the preceding claims, **characterized in that** the peptide is linked to a further substance, in particular to a labelling substance, preferably selected from the group consisting of fluorescent tags, radioactive tags, and isotope labelled groups at various positions in the amino acids.

8. A peptide according to any one of the preceding claims, **characterized in that** several peptides of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, and SEQ ID NO: 12 are covalently or non-covalently linked to each other.

9. A peptide according to any one of the preceding claims, **characterized in that** several peptides of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, and SEQ ID NO: 12 are linked to each other without a linker, i.e. directly to each other, or are linked to each other with a linker group.

10. A peptide according to any one of the preceding claims, **characterized in that** several peptides of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, and SEQ ID NO: 12 are linked together in a linear or branched manner.

11. A peptide according to any one of the preceding claims, **characterized in that** it is a dendrimer, wherein peptides of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, and SEQ ID NO: 12 are linked to a platform molecule.

12. A peptide according to any of the preceding claims, **characterized in that** it binds to the DISC1 C-region (residues 691-836) protein with a $K_D$ of less than 100 $\mu$M, in particular less than 10 $\mu$M, in particular 100 $\mu$M to 0,01 $\mu$M, more preferably 10 $\mu$M to 0,05 $\mu$M.

13. A peptide according to any of the preceding claims, **characterized in that** it binds to the clinically identified mutant version of the DISC1 C-region (residues 691-836) protein S704C, and/or the clinically identified mutant version of the DISC1 C-region (residues 691-836) protein L807-FS, and/or the clinically identified DISC1 C-region (residues 691-836) mutant version S704C with an affinity of less than 10 $\mu$M, in particular less than 1 $\mu$M, in particular 10 $\mu$M to 0,001 $\mu$M, more preferably 1 $\mu$M to 0,005 $\mu$M.

14. A peptide according to any one of the preceding claims for use as a probe for the identification, quantitative and/or qualitative determination of DISC1 and/or clinically identified mutant version of the DISC1 protein S704C, and/or the clinically identified mutant version of the DISC1 protein L807-FS, and/or the clinically identified DISC1 mutant version S704C.

15. A peptide according to at least one of claims 1 to 13 for use in the treatment of chronic mental disorders, in particular schizophrenia, major depressive disorders (MDD), and bipolar disorders (BD), and autism.

16. Use of a peptide according to at least one of the preceding claims 1 to 13 as a probe for the identification, quantitative and/or qualitative determination of the DISC1 protein S704C, and/or the clinically identified mutant version of the DISC1 protein L807-FS, and/or the clinically identified DISC1 mutant version S704C in particular as a tracer in an imaging method for clinical evaluation and/or monitoring of disease progression and/or for the early differential diagnosis of chronic mental disorders, in particular schizophrenia, major depressive disorders (MDD), and bipolar disorders (BD), and autism.

Phage Display Selection

Fig. 1

**Fig. 2**

**Fig. 3**

**Fig. 4**

Fig. 5

Fig. 6

**Fig. 7**

**Fig. 8**

Fig. 9

**Fig. 10**

Fig. 11

Fig. 12

Fig. 13

Fig. 14

Flo11: TSGVPWWKFQQQVRRT-NH2

| | |
|---|---|
| ○ | S713E |
| ○ | S704C |
| △ | L807FS |
| ▽ | WT |

**Fig. 15**

**Fig. 16**

# EUROPEAN SEARCH REPORT

Europäisches Patentamt
European Patent Office
Office européen des brevets

**Application Number**

EP 22 18 0453

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2005/255500 A1 (SAWA AKIRA [US]) 17 November 2005 (2005-11-17) * the whole document * | 1-16 | INV. C07K7/08 A61K38/04 |
| Y | CUKKEMANE A ED - FINEBERG N A ET AL: "P.675 Comprehending structural and functional attributes of the C-region of DISC1, a potential therapeutic target for schizophrenia and related mental illnesses", EUROPEAN NEUROPSYCHOPHARMACOLOGY, ELSEVIER SIENCE PUBLISHERS BV , AMSTERDAM, NL, vol. 40, 1 November 2020 (2020-11-01), XP086353769, ISSN: 0924-977X, DOI: 10.1016/J.EURONEURO.2020.09.498 [retrieved on 2020-11-17] * the whole document * | 1-16 | |
| Y | YERABHAM ANTONY S. K. ET AL: "Biophysical insights from a single chain camelid antibody directed against the Disrupted-in-Schizophrenia 1 protein", PLOS ONE, vol. 13, no. 1, 11 January 2018 (2018-01-11), page e0191162, XP055979515, DOI: 10.1371/journal.pone.0191162 Retrieved from the Internet: URL:https://journals.plos.org/plosone/article/file?id=10.1371/journal.pone.0191162&type=printable> * the whole document * | 1-16 | **TECHNICAL FIELDS SEARCHED (IPC)** C07K A61K |

-/--

The present search report has been drawn up for all claims

| Place of search | Date of completion of the search | Examiner |
|---|---|---|
| Munich | 9 November 2022 | Pinheiro Vieira, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 22 18 0453

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CUKKEMANE ABHISHEK ET AL: "Conformational heterogeneity coupled with [beta]-fibril formation of a scaffold protein involved in chronic mental illnesses", TRANSLATIONAL PSYCHIATRY, vol. 11, no. 1, 1 December 2021 (2021-12-01), XP055979520, DOI: 10.1038/s41398-021-01765-1 Retrieved from the Internet: URL:https://www.nature.com/articles/s41398-021-01765-1.pdf> * the whole document * ----- | 1-16 | |
| Y | MURDOCH H. ET AL: "Isoform-Selective Susceptibility of DISC1/Phosphodiesterase-4 Complexes to Dissociation by Elevated Intracellular cAMP Levels", THE JOURNAL OF NEUROSCIENCE, vol. 27, no. 35, 29 August 2007 (2007-08-29), pages 9513-9524, XP055979524, US ISSN: 0270-6474, DOI: 10.1523/JNEUROSCI.1493-07.2007 * the whole document * ----- | 1-16 | |

TECHNICAL FIELDS
SEARCHED    (IPC)

The present search report has been drawn up for all claims

| Place of search | Date of completion of the search | Examiner |
|---|---|---|
| Munich | 9 November 2022 | Pinheiro Vieira, E |

EPO FORM 1503 03.82 (P04C01)

## ANNEX TO THE EUROPEAN SEARCH REPORT
## ON EUROPEAN PATENT APPLICATION NO.

EP 22 18 0453

This annex lists the patent family members relating to the patent documents cited in the above-mentioned European search report.
The members are as contained in the European Patent Office EDP file on
The European Patent Office is in no way liable for these particulars which are merely given for the purpose of information.

09-11-2022

| Patent document cited in search report | Publication date | Patent family member(s) | Publication date |
|---|---|---|---|
| US 2005255500 A1 | 17-11-2005 | JP 4948768 B2 | 06-06-2012 |
| | | JP 2005245437 A | 15-09-2005 |
| | | US 2005255500 A1 | 17-11-2005 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SMITH, T. F. ; WATERMAN, M. S.** *Adv. Appl. Math.,* 1981, vol. 2, 482-489
- **NEEDLEMAN, S. B. ; WUNSCH, C. D.** *J. Mol. Biol.,* vol. 48, 443-453
- Römpp Chemie Lexikon. Georg Thieme Verlag, vol. 1, 650
- **CUKKEMANE A ; BECKER N ; ZIELINSKI M ; FRIEG B ; LAKOMEK NA ; HEISE H ; SCHRODER GF ; WILLBOLD D ; WEIERGRÄBER OH.** Conformational heterogeneity coupled with beta-fibril formation of a scaffold protein involved in chronic mental illnesses. *Transl. Psychiatry,* 2021, vol. 11, 639
- **HASHIMOTO R ; NUMAKAWA T ; OHNISHI T ; KUMAMARU E ; YAGASAKI Y ; ISHIMOTO T ; MORI T ; NEMOTO K ; ADACHI N ; IZUMI A.** Impact of the DISC1 Ser704Cys polymorphism on risk for major depression, brain morphology and ERK signaling. *Hum Mol Genet,* 2006, vol. 15, 3024-3033
- **SACHS NA ; SAWA A ; HOLMES SE ; ROSS CA ; DELISI LE ; MARGOLIS RL.** A frameshift mutation in Disrupted in Schizophrenia 1 in an American family with schizophrenia and schizoaffective disorder. *Mol Psychiatry,* 2005, vol. 10, 758-764
- **ISHIZUKA K ; KAMIYA A ; OH EC ; KANKI H ; SESHADRI S ; ROBINSON JF ; MURDOCH H ; DUNLOP AJ ; KUBO K-I ; FURUKORI K.** DISC1-dependent switch from progenitor proliferation to migration in the developing cortex. *Nature,* 2011, vol. 473, 92-96
- **YERABHAM ASK ; MAS PJ ; DECKER C ; SOARES DC ; WEIERGRÄBER OH ; NAGEL-STEGER L ; WILLBOLD D ; HART DJ ; BRADSHAW NJ ; KORTH C.** A structural organization for the Disrupted in Schizophrenia 1 protein, identified by high-throughput screening, reveals distinctly folded regions, which are bisected by mental illness-related mutations. *J. Biol. Chem.,* 2017, vol. 292, 6468-6477
- **KREJCI A ; HUPP TR ; LEXA M ; VOJTESEK B ; MULLER P.** Hammock: a hidden Markov model-based peptide clustering algorithm to identify protein-interaction consensus motifs in large datasets. *Bioinformatics,* 2016, vol. 32, 9-16
- **KORTH C.** Aggregated proteins in schizophrenia and other chronic mental diseases: DISC1opathies. *Prion,* 2012, vol. 6 (2), 134-41